Europäisches Patentamt

**European Patent Office**

**Office européen des brevets**

(11) Publication number: **0 198 651**
A2

## EUROPEAN PATENT APPLICATION

(21) Application number: **86302582.1**

(22) Date of filing: **08.04.86**

(51) Int. Cl.⁴: **A 61 M 1/06**

(30) Priority: **12.04.85 GB 8509419**

(43) Date of publication of application: **22.10.86**
**Bulletin 86/43**

(84) Designated Contracting States: **AT BE CH DE FR GB IT LI LU NL SE**

(71) Applicant: **FGL PROJECTS LIMITED, 26 Three Kings Yard Davies Street, London W1Y 1FL (GB)**

(72) Inventor: **Gannon, Raymond, c/o FGL Projects Limited 26 Three Kings Yard, Davies Street London W1Y 1FL (GB)**

(74) Representative: **Wharton, Peter Robert et al, Urquhart-Dykes & Lord Alliance House, 29-31 Kirkgate Bradford West Yorkshire, BD1 1QB (GB)**

(54) **An apparatus for applying a vacuum to a part of a human or animal body.**

(57) An apparatus, particularly a breast pump, for the application of a vacuum to a part of a human or other animal body comprising a cup member and a flexible diaphragm made preferably of a silicone rubber and fitted over the mouth of the cup member. The diaphragm has an aperture around which there is preferably a tubular extension directed inwardly of the cup member. The diaphragm is provided also preferably with a return flange to enable the diaphragm to be connected detachably to the cup member. A vacuum is provided either by deforming a wall of the apparatus or preferably by a vacuum pump which communicates with an aperture in the apparatus.

ACTORUM AG

## AN APPARATUS FOR APPLYING A VACUUM TO A PART OF A HUMAN OR ANIMAL BODY

This invention relates to apparatus for applying a vacuum to a part of a human or animal body.

Although the present invention is primarily directed to any novel integer or step, or combination of integers or steps, herein disclosed and/or as shown in the accompanying drawings, nevertheless, according to one particular aspect of the present invention to which, however, the invention is in no way restricted, there is provided an apparatus for applying a vacuum to a part of a human or animal body comprising; a cup member, means for creating a vacuum within the interior of the member and a flexible diaphragm forming a cover to the cup member and having an aperture therein, the arrangement being such that, in use, the diaphragm is positioned so that a vacuum can be applied to the said part of the body through said aperture.

The cup member preferably has means for selectively connecting the interior of the cup member to astmosphere thereby creating a varying vacuum in the chamber to apply a pulsating action to the said part of the body.

The cup member may have outlet means to be connected to a fluid receiving receptacle so that fluid extraction from the said part of the body can be collected in the receptacle.

The diaphragm may have a tubular extension surrounding the aperture and projecting towards the interior of the cup member.

In one embodiment the apparatus is for the expression of human milk. In another embodiment the apparatus is for obstetric vacuum extraction.

The invention is illustrated, merely by way of example, in the accompanying drawings, in which:-

Figure 1 is a perspective view of an apparatus according to the present invention for the expression of human milk.

Figure 2 is a cross-sectional view of a breast pump cup of the apparatus for figure 1.

Figure 3 is a perspective view of a breast contact diaphragm of the apparatus of figure 1.

Figure 4 is a cross-sectional view of an apparatus according to the present invention for obstetric vacuum extraction.

Figure 5 shows in vertical section an exploded side view of an alternative form of apparatus according to the present invention.

Figures 6 and 7 show views taken in perspective of other forms of the apparati.

Figures 8 and 9 show side views taken in vertical section of another form of the apparatus in different stages of its operation.

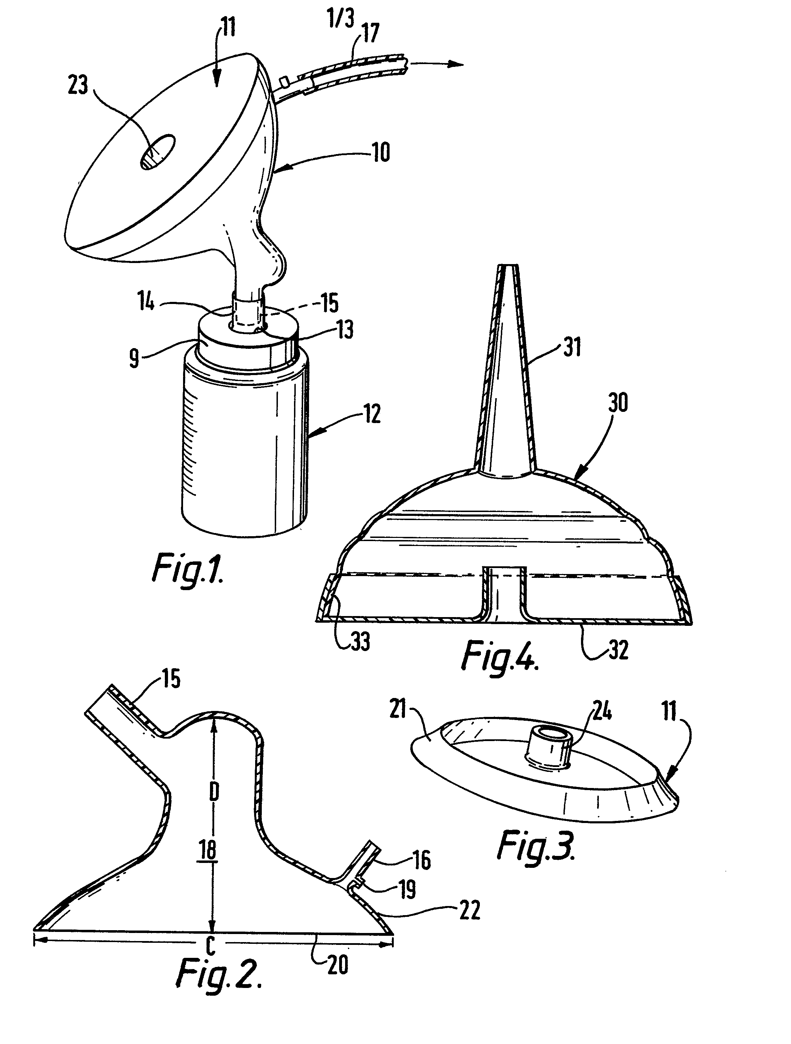

Referring first to figure 1 there is shown an apparatus according to the present invention for the expression of human milk (mother's milk). The apparatus essentially comprises a rigid breast cup member (10), made for example of clear, shatterproof and sterilisable material such as an acrylic or polycarbonate plastics material, and a breast contact diaphragm (11) which flexibly grips over a rim of the cup member (10) but is removable. The apparatus is, in use, connected with a conventional feeding bottle (12). A flexible flange (13) sealingly retained on the feeding bottle (12) by an apertured cap (9) screw-threaded onto the feeding bottle, has an integral tubular extension (14) whose size and shape is such that it forms a seal with a cylindrical outlet (15)

0198651

of the cup member (10).

The cup member (10) is shown in greater detail in figure 2. In addition to the outlet (15), the cup member (10) has an outlet (16) which allows flexible tubing (17) (figure 1) to connect the interior (18) of the cup member to a source of vacuum (not shown). The vacuum source may be a vacuum pump or a venturi pump of other equipment which will produce an appropriate reduced pressure in the interior (18) of the cup member in use. Branched from the outlet (16) is a small inlet (19) which allows air to be admitted into the cup member in a restricted, continuous or pulsed manner as will be described in greater detail hereinafter. The cup member has an open end (20) with a diameter C which is preferably between 50 mm and 110 mm. The depth D of the cup member may be between 60 mm and 70 mm. The internal diameter of the outlet (15) may be 14 mm and the internal diameter of the outlet (16) may be 9 mm. The tube (19) may have an internal diameter of 3 mm.

The breast contact diaphragm (11) is shown in greater detail in figure 3. The diaphragm (11) is made of resilient highly flexible material such as, for example, latex, silicone rubber or other elastomeric material. An integral return flange (21) of the diaphragm (11) enables it to be stretched over the open end (20) of the cup member and to grip flexibly over an exterior flanged rim (22) thereof. The diaphragm (11) has a central aperture (23), for example, with a diameter of substantially 15 mm, and an integral tubular extension (24) which, when the diaphragm is fitted onto the cup member projects into the interior (18) thereof.

In use, the cup member with the diaphragm (11) thereon is connected to the feeding bottle (12) by inserting the outlet (15) of the cup member into the tubular extension (14) ensuring that there is sealing engagement therebetween. The tubing (17) is then connected between the outlet (16) and the source of vacuum. The apparatus is positioned so that the mother's nipple enters the tubular extension (24) of the diaphragm (11) which effects a seal with the nipple protruding into the interior (18) of the cup member. When the source of vacuum is applied and the tube (19) is closed, for example by the mother's finger, the nipple will be drawn gently, but firmly, to the rear of the cup member. By opening and closing inlet (19) with her finger, the mother can create a varying vacuum within the cup member so applying a pulsating action to the breast and nipple to stimulate milk expression. The human milk so expressed passes through the outlet (16) and collects in the feeding bottle (12). As an alternative, a slowly reciprocating, self-releasing valve arrangement (not shown) could be fitted to inlet (19) to create the varying vacuum within the cup member. Furthermore if desired outlet (16) and inlet (19) can form part of feeding bottle (12) rather than a part of cup member (10).

The apparatus illustrated in figure 3 has the advantage that when the pulsating action is created by the varying vacuum within the cup member, the diaphragm simulates, almost exactly, the shape, pull and suck of a baby's mouth when being breast fed,

Conventional breast pumps which have a rigid open cup which can be evacuated, have a diameter at the rim of about 65 mm so that a hard rim is offered to the breast to effect a seal. The majority of mothers who require a breast pump do so because their breasts are too sore or too tender to allow the baby to be breast fed. The hard rim may, therefore, be a further aggravation. To overcome this problem the cup member of the apparatus of figures 1 to 3 can be made in a single size to accommodate most, if not all, sizes of breast, thus together with the flexible diaphragm making the apparatus more comfortable to use.

Figure 4 shows an apparatus according to the present invention for obstetric vacuum extraction used as an alternative to the forceps delivery techique. A cup member (30) has an integral tube (31) for attachment by flexible tubing (not shown) to a source of vacuum. A diaphragm (32) having the construction illustrated in figure 3, is fitted over and resiliently grips a rim (33) of the cup member (30). The contour following flexibility of the diaphragm (32) reduces the need for the high levels of vacuum normally needed to compensate leakage at the rim of conventional cups used for obstetric vacuum extraction. This, therefore, reduces scalp abrasions and cephalohematoma normally associated with conventional vacuum extraction cups.

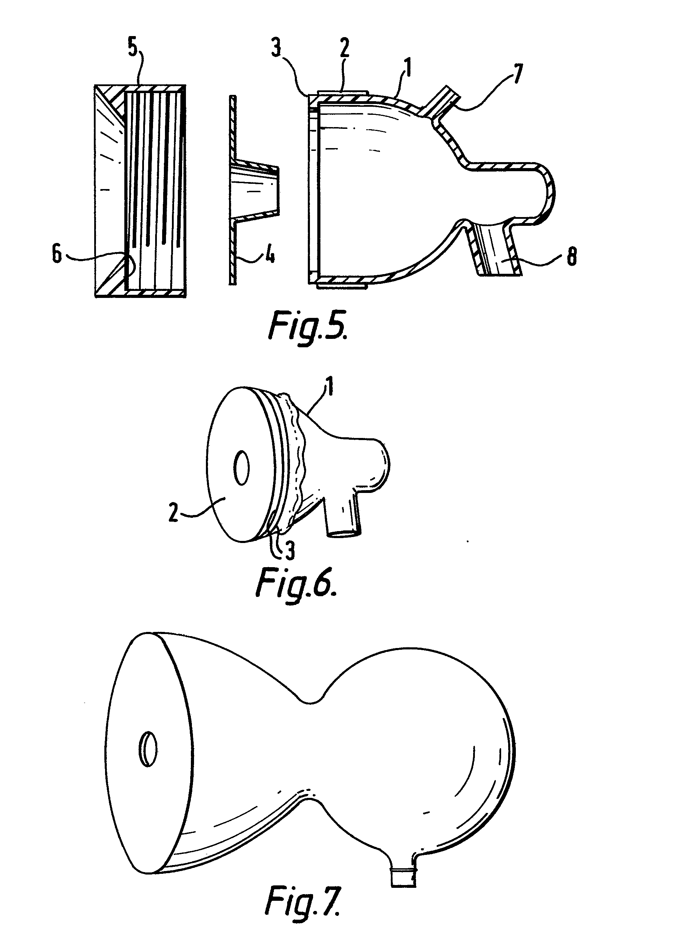

The diaphragm can be incorporated with the apparatus in different ways. The preferred method is to employ a separate diaphragm which is made of material which is both flexible and resilient and to fit the diaphragm over the rim of the cup in the manner shown in figures 1 and 2. However other methods can be used. For example, the cup member and the diaphragm can be combined integrally with one another. Alternative methods are illustrated in figures 5 to 8 below.

In figure 5 a cup member (1) is threaded and has a rim (2) terminating in a flange (3) against which a flexible diaphragm (4) abuts. The diaphragm is maintained in position by threaded member (5) having an internal shoulder (6). When member (5) is screwed tightly onto the threaded member (2) diaphragm (4) squeezed between shoulder (6) and flange (3) to form an airtight connection. A vacuum can be created in cup (1) by connecting aperture (7) to a source of vacuum (not shown) and liquid collecting in the cup can be transferred to a receptacle (not shown) in airtight connection with outlet (8) by tipping the cup so that the liquid overflows through the outlet.

In figure 6 a cup member (1) is provided with one or more grooves. A flexible diaphragm (2) is fitted over the mouth of the cup member and is maintained in position by one or more elastic rings which force the edge of the diaphragm sufficiently into the grooves so as to form an airtight connection between the outer wall of the cup and the diaphragm.

A vacuum can be created in the cup member either by connecting the cup member or the receptacle to which it is attached to an external source of vacuum, for example, a pump which is available under the trade mark VAC SAC. A very satisfactory source is the subject of our copending patent application no. 8516838 which is directed to a vacuum device comprising a by-pass vacuum pump, a conduit connecting the pump to an applicator head and a means enabling a part of the stream of air entering the pump to by-pass the applicator head. A venturi pump can be used as the by-pass means.

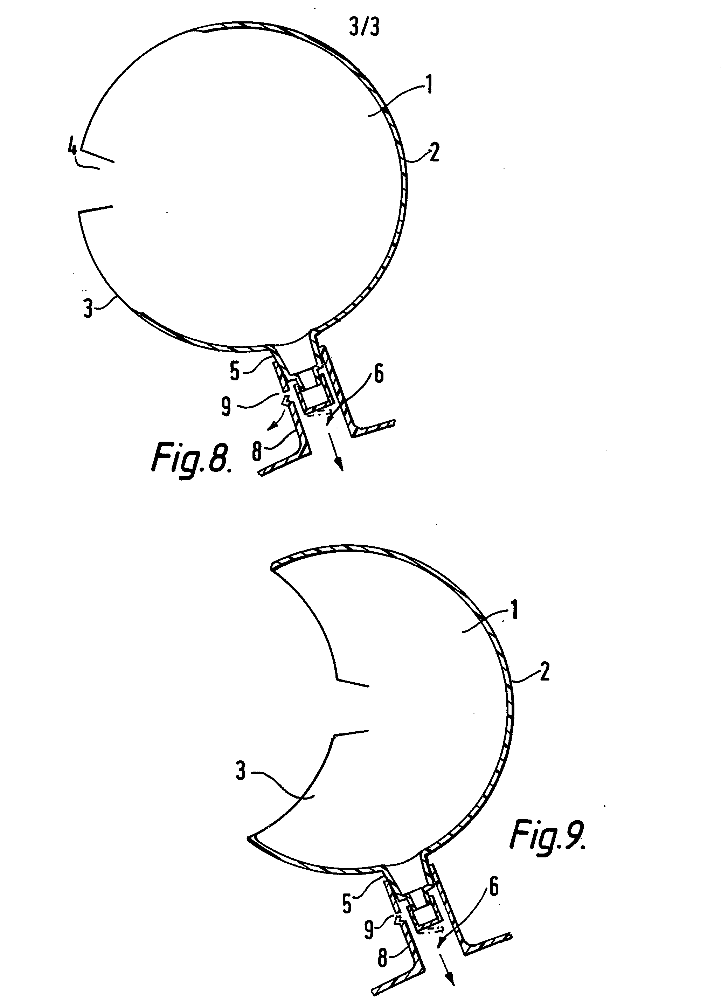

A further example of a pump according to the present invention in which a vacuum is created internally by manual means is illustrated in figures 8 and 9. In these figures chamber (1) is a potential cup member. It has a roughly spherical shape and a diameter of about 3.5". Approximately two thirds of its wall surface is made of resiliently deformable material and is thicker than the remaining one third of the wall. The thinner wall (3) is readily deformable and is provided with an aperture (4) for receiving a nipple. The two wall sections may be made separately and joined together to form a vessel or the vessel can be made in a single moulding operation. The vessel is provided with a neck (5) to which is connected a flap non-return valve (6). Fitted onto the neck is a rubber connector for connecting the device to a receptacle which conveniently can be a baby's feeding bottle. The rubber connector is provided with vent (9).

In operation the nipple of the user is presented to the aperture (4) and the apparatus is pressed gently against the breast. At this stage wall (3) is deformed in the direction of wall (2) and because of its considerable flexibility wall (3) conforms readily to the shape of the breast without causing discomfort to the user. Wall (2) is then first compressed and then slowly allowed to expand thus creating a vacuum within the vessel. Milk is abstracted and collects at the bottom of the vessel. At this stage wall (2) is compressed once again causing the milk to discharge into receptacle through valve (6) and air to discharge from vent (9). When the pressure is releasd from wall (2) a vacuum is created once more in the vessel. Valve (6) closes and the process of milk abstraction begins once again.

The form of pump described in figures 8 and 9 can be adapted readily for operation with an external vacuum pump. For this purpose wall (2) is preferably of rigid or semi rigid construction and is provided with an aperture for connection to a vacuum pump. Drainage of milk into the receptacle can be effected without the need for either non-return valve (6) or vent (9) shown in the previous two drawings.

An apparatus according to the present invention has other uses. For example, apparatus similar to that shown in figure 1 may be used for collecting urine from, for example, bed-ridden patients. To this end the feeding bottle (12) is replaced by a urine collecting receptacle and the diaphragm modified to provide a slit aperture in place of the aperture (23) and the tubular extension (24). In operation, the penis of a male patient would be inserted through the slit aperture and the application of vacuum would cause the diaphragm to engage sealingly the penis and allow the urine to be collected in the urine collecting receptacle.

Apparatus according to the present invention may also be used to remove faeces from bed-ridden, especially geriatric, patients and for the drainage of cysts and wounds. Apparatus according to the present invention is not restricted to use on the human body but may, if desired, have veterinary uses, for example, obstetric vacuum extraction on animals, draining of cysts and wounds etc.

One major advantage of the apparatus according to the present invention and described above is that the diaphragm when separate from the rest of the apparatus is relatively inexpensive and may be disposed of after it has been used once or a number of times. This is very beneficial from the point of view of hygiene.

## CLAIMS

1. An apparatus for applying a vacuum to a part of a human or other animal body comprising a cup member, means for creating a vacuum within the interior of the member and a flexible diaphragm forming a cover for the cup member and having an aperture therein, the arrangement being such that in use the diaphragm is positioned so that a vacuum can be applied to the part of the body through the aperture.

2. An appartus according to claim 1 wherein the diaphragm is connected detachably to the cup member.

3. An apparatus according to either claims 1 or 2 wherein the diaphragm grips resiliently the cup member.

4. An apparatus according to any one of the preceding claims wherein the diaphragm incorporates a tubular extension which projects into the cup member.

5. An apparatus according to any one of the preceding claims wherein the diaphragm is made of elastomeric material.

6. An apparatus according to claim 5 wherein the

0198651

elastomeric material is a silicone.

7.      An apparatus according to any one of the preceding claims wherein the diaphragm incorporates an integral return flange.

8.      An apparatus according to any one of the preceding claims wherein the means for creating a vacuum is a resiliently deformable wall forming part of the apparatus.

9.      An apparatus according to any one of claims 1 to 5 wherein the means for creating a vacuum comprises an aperture for communication with an electric pump.

10.     An apparatus according to any one of claims 1 to 7 and 9 incorporating an inlet by which the opening and closing thereof enables the vacuum within the apparatus to be varied.

0198651

**Fig.1.**

**Fig.2.**

**Fig.3.**

**Fig.4.**

Fig.5.

Fig.6.

Fig.7.

3/3

Fig.8.

Fig.9.